Europäisches Patentamt

(19) European Patent Office

Office européen des brevets

(11) Publication number: **0 127 389**
**B1**

(12) EUROPEAN PATENT SPECIFICATION

(45) Date of publication of patent specification: **07.09.88**

(51) Int. Cl.⁴: **A 61 K 31/415**

(21) Application number: **84303335.8**

(22) Date of filing: **17.05.84**

(54) N,N-diethyl-5-methyl-2H-1-benzothiopyrano-(4,3,2-cd)-indazole-2-ethanamine, compositions comprising the same and uses therefor.

(30) Priority: **20.05.83 US 496612**

(43) Date of publication of application:
**05.12.84 Bulletin 84/49**

(45) Publication of the grant of the patent:
**07.09.88 Bulletin 88/36**

(84) Designated Contracting States:
**AT BE CH DE FR GB IT LI LU NL SE**

(56) References cited:

**JOURNAL OF MEDICINAL CHEMISTRY, vol. 6,
March 1963, pages 185-191, Washington, US;
E.J. BLANZ et al.: "A systematic investigation
of thioxanthen-9-ones and analogs as potential
antitumor agents"**

The file contains technical information
submitted after the application was filed and
not included in this specification

(73) Proprietor: **WARNER-LAMBERT COMPANY
201 Tabor Road
Morris Plains New Jersey 07950 (US)**

(72) Inventor: **Elslager, Edward Faith
4081 Thornoaks Drive
Ann Arbor Michigan 48104 (US)**
Inventor: **Werbel, Leslie Morton
1570 Covington Drive
Ann Arbor Michigan 48103 (US)**

(74) Representative: **Jones, Michael Raymond et al
HASELTINE LAKE & CO. Hazlitt House 28
Southampton Buildings Chancery Lane
London WC2A 1AT (GB)**

## Description

This invention is directed to the use of N,N - diethyl - 5 - methyl - 2*H* - 1 - benzothiopyrano - [4,3,2 - cd] - indazole - 2 - ethanamine or a pharmaceutically acceptable salt thereof, in the manufacture of a medicament for use as an anti-tumor agent in warm blooded animals.

The chemical compounds described as 5-methyl, hydroxymethyl, or 5-formyl benzothiopyranoindazole compounds, including N,N - diethyl - 5 - methyl - 2*H* - 1 - benzothiopyrano - [4,3,2 - cd] - indazole - 2 - ethanamine, and their use as antiparasitic and antibacterial agents, are known from US—A—3,505,341. However, as a class, the compounds lack antitumor properties and their use in this context has therefore understandably been disregarded.

The Journal of Medicinal Chemistry, Vol. 6, March 1963, pages 185—191 (E. J. Blanz, et al) discloses certain thioxanthen - 9 - ones and thioxanthen - 9 - one anologues, including 2H - (1)benzothiopyrano (4,3,2 - cd) indazole, which are reported to posses anti-tumor activity.

EP—A—0114002 discloses substituted benzothiopyrano[4,3,2 - cd] - indazoles, methods for their production, pharmaceutical compositions comprising the compounds, and methods of treatment using the compounds in dosage form. The compounds have pharmacological properties and are useful antibacterial agents, antifungal agents, and antitumor agents.

Regarding tumor disease, control of malignant tumors in man and animals still remains as an unrealized goal. Within the last several decades, understanding of malignancy has made significant progress; however, conquering of the malignant disease state has not been realized.

Conventional therapy of both humans and other valuable animal species inflicted with malignant tumors presently includes surgical excising of the tumor, local radiation therapy of the afflicted animal, and chemotherapy by administration of a chemotherapeutic agent to the animal. The death of a significant number of patients inflicted with malignant tumors is attributable not to the primary tumor but instead to metastasis of the primary tumor to secondary sites in the host. If a primary tumor is detected early, it normally can be eliminated by surgery, radiation or chemotherapy, or combinations of these. The metastatic colonies of these primary tumors, however, are harder to detect and eliminate and the unsuccessful management of them remains a serious medical problem.

Tumors are normally classified either as benign or malignant. The malignant tumor is characterized by its ability to invade both surrounding tissue and to colonize distant sites via metastasis. Certain organs are more prone to metastatis than others. Included in this group are the lung, the brain, the liver, the ovaries, and the adrenal glands. It has further been suspected that both surgery and radiation of a primary tumor in certain instances actually promote metastasis.

In view of the inability of current cancer therapy to successfully control the malignant tumor and its metastases, a need for additional chemotherapeutic agents exists.

The present invention is based on the unexpected finding that N,N - diethyl - 5 - methyl - 2*H* - 1 - benzothiopyrano - [4,3,2 - cd] - indazole - 2 - ethanamine, sometimes referred to hereinafter as Compound I, uniquely possesses outstanding antitumor properties.

In a first aspect of the present invention, there is disclosed the use of N,N - diethyl - 5 - methyl - 2*H* - 1 - benzothiopyrano - [4,3,2 - cd] - indazole - 2 - ethanamine or a pharmaceutically acceptable salt thereof, in the manufacture of a medicament for use as an anti-tumor agent.

According to a second aspect of the present invention, there is disclosed the use of a pharmaceutical composition in the manufacture of a medicament for use as an anti-tumor agent, which composition comprises an active ingredient and a pharmaceutically acceptable carrier, wherein the active ingredient is N,N - diethyl - 5 - methyl - 2*H* - 1 - benzothiopyrano - [4,3,2 - cd] - indazole - 2 - ethanamine in free base form or in a pharmaceutically acceptable salt form.

Pharmaceutically acceptable salt forms include: the sulphate, the phosphate, the hydrochloride, the sulphamate, the ethanesulphonate, the isethionate, the benzenesulphonate, the p-toluenesulphonate, the lactate, the gluconate, the citrate or the glucuronate, all being suitable for use in the treatment and inhibition of malignant tumours in warm blooded animals.

When being utilized as antitumor preparations, the pharmaceutical compositions can take any of a wide variety of parenteral dosage forms. The dosage forms may comprise, as the active component, Compound I as the free base and/or corresponding pharmaceutically acceptable salt or salts, such as the sulfate, phosphate, or methanesulfonate salt. Other appropriate pharmaceutically acceptable salts are those derived from mineral acids such as hydrochloric acid and sulfamic acid; and organic acids such ethanesulfonic acid, benzenesulfonic acid and p-toluenesulfonic acid, giving the hydrochloride, sulfamate, ethanesulfonate, isethionate, benzenesulfonate, p-toluenesulfonate, lactate, gluconate, citrate, and glucuronate. A preferred salt is the methanesulfonate.

The acid addition salts are prepared either by dissolving the free base in aqueous or aqueous alcohol solution or other suitable solvents containing the appropriate acid and isolating the salt by evaporating the solution, or by reacting the free base and acid in an organic solvent, in which case the salt separates directly or can be obtained by concentration of the solution.

For preparing pharmaceutical compositions, one uses inert, pharmaceutically acceptable carriers that can be either solid or liquid. Solid form preparations include ointments and suppositories. A solid carrier can be one or more substances which may also act as diluents, lubricants, suspending agents, or binders. Suitable solid carriers are polyethylene glycol, low melting wax, and cocoa butter.

Liquid form preparations include solutions, suspensions, and emulsions. As an example may be mentioned water or water-propylene glycol solutions for parenteral injection. Liquid preparations can also be formulated in solution in aqueous polyethylene glycol solution.

Preferably, the pharmaceutical preparation is in unit dosage form. In such form, the preparation is subdivided into unit doses containing appropriate quantities of the active component. The unit dosage form can be a packaged preparation, for example, vials or ampoules. The package containing discrete quantities of preparation.

The quantity of active compound in a unit dose of liquid preparation may be varied or adjusted in the range from 0.1 mg to 500 mg according to the particular application and the potency of the active ingredient; the range from 10 to 100 mg/ml of carrier is preferred. The daily parenteral doses for mammalian subjects to be treated ranges from 0.1 mg/kg to 100 mg/kg. The preferred daily dosage range is 1.0 mg/kg to 10 mg/kg.

The pharmaceutical compositions, as indicated, are constituted so that they can be administered parenterally. Solutions of the active compound as a free base or pharmaceutically acceptable salt can be prepared in water suitably mixed with a surfactant such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol), suitable mixtures thereof and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, and thimerosal. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of the sterile powders for the preparation of sterile injectable solutions, the preferred methods of preparation are vacuum drying and the freeze-drying technique which yield a powder of the active ingredient plus any additional desired ingredient from a previously sterile-filtered solution thereof.

As used herein, "pharmaceutically acceptable carrier" includes any and all solvents, dispersion media, coatings, antibacterial and antifungal agents, isotonic and absorption delaying agents. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions.

The pharmaceutical compositions preferably comprise, as the active component, at least 0.1 percent by weight, based on the total weight of the composition, of N,N - diethyl - 5 - methyl - 2$H$ - 1 - benzothiopyrano - [4,3,2 - cd] - indazole - 2 - ethanamine in free base form or a pharmaceutically acceptable salt form.

It is especially advantageous to formulate parenteral compositions in dosage unit form for ease of administration and uniformity of dosage. Dosage unit form as used herein refers to physically discrete units suitable as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitation inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore disclosed. The usefulness of the pharmaceutical compositions of the present invention as antitumor preparations

3

**0 127 389**

is demonstrated by the effectiveness of Compound I in standard antitumor activity test procedures, both in vivo and in vitro.

Test for in vivo antileukemic activity

The in vivo lymphocytic leukemia P388 test is carried out by the United States National Cancer Institute. The animals used are either male or female $CD_2F_1$ mice. There are six to seven animals per test group. The tumor transplant is by intraperitoneal injection of dilute ascitic fluid containing cells of lymphocytic leukemia P388. The test compounds are administered intraperitoneally in two single doses with a four-day interval between doses at various dose levels following tumor inoculation. The animals are weighed and survivors are recorded on a regular basis for 30 days. A ratio of survival time for treated (T)/control (C) animals is calculated. The criterion for efficacy is T/C×100>125%. The positive control compound in this test is 1,4 - dihydroxy - 5,8 - [bis[2 - [(2 - hydroxyethyl)amino] - ethyl]amino] - 9,10 - anthracenedione given at dosages ranging from 12.0 to 0.075 mg/kg. See *Cancer Chemotherapy Reports*, Part 3, *3*, 1 (1972) for a comprehensive discussion of the protocol.

Utilizing this procedure, the following results were obtained.

TABLE 1

Compound I, methanesulfonate

| Dose mg/kg | T/C×100 |
| --- | --- |
| 25 | 188; 215 |
| 12.5 | 136; 157 |

TABLE 2

Broad spectrum antitumor activity* of compound I, methanesulfonate

| Tumor | Route*** Tumor/ drug | Regimen | Dose mg/kg | T/C×100 (percent) | |
| --- | --- | --- | --- | --- | --- |
| B16 Melanoma (B6C3F1) | IP/IP | Q01D×09 | 6.25 | 135 | |
| Colon 38** (BDF1) | SC/IP | Q07D×02 | 100 50 | 16 37 | (One cure) |
| Mammary** CD8F1 | SC/IP | Q07D×05 | 100 50 | 20 13 | |
| Lewis lung (BDF1) | IV/IP | Q01D×09 | 25 | 156 | (3/10 cures) |

*Procedure described in Cancer Chemotherapy Reviews, *7*, 167 (1980) and references cited therein.
**For these tumors median tumor weight is evaluated versus controls rather than median survival time and T/C is the percentage of the tumor weight in the treated animals relative to the controls.
***IP=Intraperitoneal
SC=Subcutaneous
IV=Intravenous

Test for in vitro activity against human solid tumors

HCT-8 (human colon adenocarcinoma) cells are trypsinized using Trypsin-EDTA. A single cell suspension is achieved by passing the cells through a 26 gauge needle with a 20 ml syringe. A cell suspension is prepared using RPMI 1640+10% FCS+50% μg/ml garamycin with a cell concentration of approximately 30,000 cells/ml. The cell suspension is dispensed in Linbro® 24-well plates; 1 ml/well. The plates are incubated for approximately 48 hours at 37°C in a 5% $CO_2$ atmosphere. At this time test compounds are added in the appropriate concentration. Five μl of the 200 μg/ml stock solution is added to each well in a primary test. Ten μl of the appropriate dilution is added to each well for a titration test. The plates are reincubated an additional 60—65 hours at 37°C in a 5% $CO_2$ atmosphere. The test is read by lysing the cells using a mix of cationic surfactant, glacial acetic acid and sodium chloride. Two ml of the lysed cell suspension from each well is added to 8 ml of diluent. Each sample is read on the Coulter® counter (ZBI model). The activity of each sample is measured as a percentage of the controls and the data is reported as $ID_{50}$, that is, the concentration of drug required to kill 50% of the tumor cells.

Utilizing this procedure, the result for Compound I methanesulfonate is $ID_{50}=1.1×10^{-6}M$.

4

Clonogenic assay against human tumors

Compound I has also been examined against primary human tumor cultures in the clonogenic assay. A description of the test can be found in A. W. Hamburger and S. E. Salmon, *Science*, 197,461—3(1977). The results of this test have been shown to correlate with human in vivo response; see, for example, P. A. Johnson and A. H. Rossof, *Arch. Intern. Med., 143*, 111—114 (1983). Thus, Compound I evaluated at 10 µg/ml continuous exposure against fresh human breast, lung, and ovarian tumors resulted in a response rate (based on ≤50% survival of tumor cells) of: breast 2/5=40%; lung 4/10=40%; ovarian 2/9=22%.

The following representative examples are given as illustrative pharmaceutical compositions utilizing different carriers. In these examples, Example 1 illustrates the use of the compounds of the invention in injectables suitable for intravenous or other types of injection into the host animal. Example 2 is directed to use of the compounds of the invention in suitable suppositories. For the examples the context of Compound I is given as that of free base, and the ingredients are listed followed by the methods of preparing the compositions.

Example 1

Example 1a Compound I, hydrochloride salt

Compound I     125 mg—500 mg

Water for Injection USP q.s.

Compound I is dissolved in the water and passed through a 0.22 µm filter. The filtered solution is added to ampoules or vials, sealed and sterilized.

Example 1b Compound I, methanesulfonate salt

Compound I     125 mg—500 mg

Water for Injection USP q.s.

Prepared as per Example 1a above.

Example 2

Example 2a Compound I, methanesulfonate salt

125 mg 250 mg or 500 mg per 3 g

|  | 125 mg | 250 mg | 500 mg |
|---|---|---|---|
| Compound I | 125 mg | 250 mg | 500 mg |
| Polyethylene Glycol 1540 | 1925 mg | 1750 mg | 1400 mg |
| Polyethylene Glycol 8000 | 825 mg | 750 mg | 600 mg |

Melt the Polyethylene Glycol 1540 and the Polyethylene Glycol 8000 together at 60°C and dissolve Compound I into the melt. Mold this total at 25°C into appropriate suppositories.

Example 2b Compound I, methanesulfonate salt

125, 250, 500 mg per 3 g

|  | 125 mg | 200 mg | 250 mg |
|---|---|---|---|
| Compound I | 125 mg | 200 mg | 250 mg |
| Polyethylene Glycol 1540 | 1925 mg | 1750 mg | 1400 mg |
| Polyethylene Glycol 8000 | 825 mg | 750 mg | 600 mg |

Prepare as per Example 2a above.

**Claims**

1. The use of N,N - diethyl - 5 - methyl - 2*H* - 1 - benzothiopyrano - [4,3,2 - cd] - indazole - 2 - ethanamine or a pharmaceutically acceptable salt thereof, in the manufacture of a medicament for use as an anti-tumor agent.

2. The use of a pharmaceutical composition in the manufacture of a medicament for use as an anti-tumor agent, which composition comprises an active ingredient and a pharmaceutically acceptable carrier, wherein the active ingredient is N,N - diethyl - 5 - methyl - 2*H* - 1 - benzothiopyrano - [4,3,2 - cd] - indazole - 2 - ethanamine in free base form or in a pharmaceutically acceptable salt form.

3. For use as claimed in Claim 1 or 2, a compound, or a pharmaceutical composition, wherein the pharmaceutically acceptable salt form is the methanesulphonate salt form.

4. For use as claimed in Claim 1 or 2, a compound, or a pharmaceutical composition, wherein the pharmaceutically acceptable salt form is one of the following: the sulphate, the phosphate, the hydrochloride, the sulphamate, the ethanesulphonate, the isethionate, the benzenesulphonate, the p-toluenesulphonate, the lactate, the gluconate, the citrate or the glucuronate.

5. For use as claimed in Claim 2 or Claim 3 or 4 when appendant to Claim 2, the pharmaceutical composition in which the active ingredient is present in an amount of at least 0.1% by weight, based on the total weight of the pharmaceutical composition.

6. For use as claimed in claim 2 or any one of Claims 3, 4 and 5 when appendant to claim 2, the pharmaceutical composition in which the active ingredient is present in an amount of from 0.1 mg to 500 mg.

## Patentansprüche

1. Verwendung von N,N - Diäthyl - 5 - methyl - 2*H* - 1 - benzothiopyrano[4,3,2 - cd]indazol - 2 - äthanamin oder eines pharmazeutisch akzeptablen Salzes davon bei der Herstellung eines Medikaments zur Verwendung als Anti - Tumor - Mittel.

2. Verwendung einer pharmazeutischen Zusammensetzung bei der Herstellung eines Medikaments zur Verwendung als Anti - Tumor - Mittel, welche Zusammensetzung ein aktives Ingrediens und einen pharmazeutisch akzeptablen Träger umfaßt, worin das aktive Ingrediens N,N - Diäthyl - 5 - methyl - 2*H* - 1 - benzothiopyrano[4,3,2 - cd]indazol - 2 - äthanamin in Form der freien Base oder in Form eines pharmazeutisch akzeptablen Salzes davon ist.

3. Verbindung oder pharmazeutische Zusammensetzung zur Verwendung wie im Anspruch 1 oder 2 beansprucht, worin die pharmazeutisch akzeptable Salzform die Methansulfonatsalzform ist.

4. Verbindung oder pharmazeutische Zusammensetzung zur Verwendung wie im Anspruch 1 oder 2 beansprucht, worin die pharmazeutisch akzeptable Salzform eines der folgenden ist: das Sulfat, das Phosphat, das Hydrochlorid, das Sulfamat, das Äthansulfonat, des Isethionat, das Benzolsulfonat, das p-Toluolsulfonat, das Lactat, das Gluconat, das Citrat oder das Glucuronat.

5. Pharmazeutische Zusammensetzung zur Verwendung wie im Anspruch 2 oder Anspruch 3 oder 4 beansprucht, wenn sie auf den Anspruch 2 rückbezogen sind, worin das aktive Ingrediens in einer Menge von zumindest 0,1 Gew.-%, bezogen auf das Gesamtgewicht der pharmazeutischen Zusammensetzung, anwesend ist.

6. Pharmazeutische Zusammensetzung zur Verwendung wie im Anspruch 2 oder irgendeinem der Ansprüche 3, 4 und 5 beansprucht, wenn sie auf den Anspruch 2 rückbezogen sind, in welcher das aktive Ingrediens in einer Menge von 0,1 mg bis 500 mg anwesend ist.

## Revendications

1. L'utilisation de la N,N - diéthyl - 5 - méthyl - 2H - 1 - benzothiopyrano - [4,3,2 - cd] - indazole - 2 - éthanamine ou d'un de ses sels pharmaceutiquement acceptables pour la fabrication d'un médicament destiné à être employé comme agent antitumoral.

2. L'utilisation d'une composition pharmaceutique pour la fabrication d'un médicament destiné à être employé comme agent antitumoral, cette composition comprenant un ingrédient actif et un support pharmaceutiquement acceptable, utilisation dans laquelle l'ingrédient actif est la N,N - diéthyl - 5 - méthyl - 2H - 1 - benzothiopyrano - [4,3,2 - cd] - indazole - 2 - éthanamine sous sa forme de base libre ou sous une forme saline pharmaceutiquement acceptable.

3. Pour l'utilisation selon la revendication 1 ou 2, un dérivé ou une composition pharmaceutique dans laquelle la forme saline pharmaceutiquement acceptable est celle du méthanesulfonate.

4. Pour l'utilisation selon la revendication 1 ou 2, un dérivé ou une composition pharmaceutique dans laquelle la forme saline pharmaceutiquement acceptable est l'une des suivantes: sulfate, phosphate, chlorhydrate, sulfamate, éthanesulfonate, iséthionate, benzènesulfonate, p-toluènesulfonate, lactate, gluconate, citrate ou glucuronate.

5. Pour l'utilisation selon la revendication 2 ou la revendication 3 ou 4 dépendant de la revendication 2, la composition pharmaceutique dans laquelle l'ingrédient actif est présent en une proportion pondérale d'au moins 0,1% en poids par rapport au poids total de la composition pharmaceutique.

6. Pour l'utilisation selon la revendication 2 ou l'une quelconque des revendications 3, 4 et 5 dépendant de la revendication 2, la composition pharmaceutique dans laquelle l'ingrédient actif est présent en une quantité comprise entre 0,1 mg et 500 mg.